# EUROPÄISCHE PATENTSCHRIFT

(11) **EP 1 910 645 B1**
(45) Veröffentlichungstag und Bekanntmachung des Hinweises auf die Patenterteilung: **10.09.2014**
(21) Anmeldenummer: 06779886.8
(22) Anmeldetag: 21.07.2006
(51) Int. Cl.: F01C 1/348, F01C 13/02, F01C 20/04, F01C 20/14, F01C 21/08

(54) **MIKRO-DRUCKLUFTMOTOR**
COMPRESSED AIR MICROMOTOR
MICROMOTEUR A AIR COMPRIME

(30) Priorität: 22.07.2005 CH 12252005
(43) Veröffentlichungstag der Anmeldung: 16.04.2008
(73) Patentinhaber: Rotomed AG, 4512 Bellach (CH)
(72) Erfinder: MUELLER, Thomas, CH-4513 Langendorf (CH); MUELLER, Daniel, CH-4513 Langendorf (CH); MUELLER, Kurt, CH-4513 Langendorf (CH)
(74) Vertreter: Rosenich, Paul
(86) Internationale Anmeldenummer: PCT/IB2006/002000
(87) Internationale Veröffentlichungsnummer: WO 2007/010375

(56) Entgegenhaltungen:
- EP-A- 1 457 680
- WO-A-03/067032
- GB-A- 2 042 081
- US-A- 3 439 422
- US-A- 4 278 427
- US-A- 4 344 746
- US-A- 4 403 958
- US-A- 4 642 051

## Beschreibung

Die Erfindung betrifft einen Mikro-Druckluftmotor mit einem eine Führungsbohrung aufweisenden Gehäuse und einem darin drehbar gelagerten Rotor, wobei der Rotor mit im wesentlichen radial nach aussen verlaufenden Schlitzen versehen ist, in denen plattenförmige Lamellen durch die Fliehkraft radial nach aussen verschiebbar gelagert sind, und mit einem axial an den Rotor anschliessenden Kollektor mit zumindest je einer Ein- und Austrittsöffnung und mit äusseren Anschlüssen für die Druckluft verbundenen und in die zumindest eine Austrittsöffnung mündenden Kanälen, und mit einer Betätigungschülse wobei der Kollektor mittels der Betätigungshülse um die Drehachse des Rotors oder um eine dazu parallele Achse, bzw. um die Längsachse des Gehäuses aus einer zentralen, den Rotor stillsetzenden Neutralstellung nach beiden Seiten hin bis in eine den Rotor unter Druckluft links- bzw. rechtsdrehende Endstellung verdrehbar ist. Ein solcher Mikro-Druckluftmotor ist z.B. aus der US-A-4 403 958 bekannt.

Druckluftmotoren mit radial verschiebbaren Lamellen werden in unterschiedlicher Grösse und für ganz unterschiedliche Anwendungen gebaut und angewendet. Die als "Mikro-Druckluftmotoren" bezeichneten Geräte weisen in der Regel einen Gehäusedurchmesser von weniger als zwei Zentimeter auf und werden vorwiegend in der Medizinaltechnik, insbesondere der Chirurgie und Neurochirurgie eingesetzt. Die Mehrheit der bisher bekannten und eingesetzten Geräte kann nur in einer Drehrichtung betrieben werden. Dies hängt damit zusammen, dass die Querschnitte der Ein- und Auslassöffnungen in der Grösse meistens unterschiedlich sind, um ein optimales Drehzahl- und Leistungsverhalten zu erzielen. Sind die Ein- und Auslassöffnungen gleich gross dimensioniert, so ist zwar eine Drehrichtungsumkehr möglich, aber das Gerät erbringt in beiden Drehrichtungen nicht die optimale, sondern nur eine begrenzte Leistung.

Aus der WO 81/03520 ist ein Vakuummotor bekannt, der in beide Drehrichtungen betreibbar ist. Dabei kann der mit einer Vakuumleitung verbundene Handgriff um das Gehäuse herum nach beiden Seiten in fixe Endstellungen verdreht werden. Der Handgriff hat somit zusätzlich die Funktion eines Drehschiebers. Somit kann die eine oder andere Öffnung des Gehäuses mit der Vakuumleitung, bzw. mit der Aussenluft verbunden werden. Die Ein- und Auslassöffnungen sind jedoch somit immer gleich gross. Ein optimaler Betrieb des Motors ist somit in beiden Drehrichtungen nicht möglich.

Ein aus der GB 1 578 364 bekannter Lamellenmotor, welcher ebenfalls in der Medizlnaltechnik eingesetzt wird, weist insgesamt drei Anschlussöffnungen auf, wobei die eine Öffnung immer als Auslass und die anderen beiden Öffnungen, je nach Drehrichtung, wahlweise als Ein- bzw. Auslass dienen. Dieses Gerät weist somit in beiden Drehrichtungen ein gegenüber einem Gerät mit nur zwei Öffnungen ein verbessertes Leistungsverhalten auf. Für die Steuerung ist jedoch ein spezieller externer Schieber notwendig, der In dieser Druckschrift nicht offenbart wird.

Aus der US 4 708 210 Ist ein pneumatisch betreibbares Handwerkzeug bekannt, das einen mittels eines Betätigungsringes verdrehbaren Kollektor aufweist. Der Kollektor dient in seinen beiden Endstellungen zur Drehrichtungsumkehr des Motors. Die Drehzahl wird über ein separates Drosselventil geregelt. Diese Bauart ist aufwändig und nicht sehr benutzerfreundlich.

In der US 4 403 958 ist ein Druckluftmotor für ein zahnärztliches Werkzeug mit einem Flügelmotor geoffenbart, der einen mittels einer Betätigungshülse verdrehbaren Kollektor aufweist. Durch diesen Aufbau in Art eines durch die Betätigungshülse verstellbaren Drehschieberventils zwischen Druckluftzufuhr und Flügelmotor können die Rotationsgeschwindigkeit als auch die Drehrichtung des Motors eingestellt werden. Ähnliche Konstruktionen sind in der US 4 344 746, der US 3 439 422, der GB 2 042 081, der US 4 642 051 oder der US 4 278 427 beschrieben.

Die Erfindung sieht einen Mikro-Druckluftmotor vor, wie er in Anspruch 1 definiert wird.

Beim Kollektor handelt es sich um ein geräteinternes, praktisch bei Jedem Druckluft- oder Mikro-Druckluftmotor in unterschiedlicher Form vorhandenes Bauteil. Die Verdrehbarkeit des Kollektors kann beispielsweise auf einfache Art dadurch erreicht werden, dass der Kollektor an seiner Aussenseite im wesentlich zylindrisch ausgebildet und in einer entsprechenden Bohrung des Gehäuses verdrehbar gelagert ist.

Vorzugsweise sind in Neutralstellung des Kollektors die Räume auf beiden Seiten der Lamelle, welche der mit Druck beaufschlagten Lamelle gegenüberliegt, mit zumindest einer Austrittsöffnung verbunden.

Gemäss einer vorteilhaften Ausführungsform des Mikro-Druckluftmotors ist vorgesehen, dass zumindest eine der Eintrittsöffnungen und/oder der Austrittsöffnungen mit nierenförmigen Querschnitt und zur Drehachse des Rotors hin gekrümmt ausgeführt ist. Im Zusammenwirken mit dem meist ring-, bzw. keilförmigen Arbeitsraum des Druckluftmotors ergibt dies in allen Drehstellungen des Kollektors günstige Strömungsverhältnisse an den Übergangsstellen vom Kollektor zum Rotor.

Vorzugsweise weisen die Ein- und Austrittsöffnungen unterschiedliche Querschnitte auf. Dadurch können diese optimal den Bedürfnissen angepasst werden, dh. die Auslassöffnungen im Bereich der abströmenden Abluft können wesentlich grösser dimensioniert werden, als die Einlassöffnungen im Bereich der mit hohem Druck einströmenden Zuluft. Somit kann der Luftwiderstand über den gesamten Strömungsweg optimal gehalten werden.

Für die Steuerung, bzw. Regelung des Druckluftmotors ist es vorteilhaft, dass der Kollektor stufenlos bis zu den Endstellungen verdrehbar ist. Die Drehzahl des Druckluftmotors kann somit durch Vergrössern des Durchflussquerschnitts von Null bis zur Maximaldrehzahl praktisch stufenlos geregelt werden.

Für die Betätigung des Kollektors ist dieser zweckmässigerweise mit einer Betätigungshülse gekoppelt. Die Koppelung kann beispielsweise mittels keil-, stift-, oder schraubenförmigen Verbindungselementen erfolgen. Diese Verbindungselemente können gleichzeitig auch mit entsprechenden Führungen oder Kulissen zusammenwirken und den Verdrehweg des Kollektors steuern, bzw. begrenzen.

Da die Druckluft zum Rotor meist axial zu-, bzw. abgeführt wird, ist es vorteilhaft, dass wenigstens ein Kanal koaxial, bzw. parallel zur Achse des Rotors verläuft. Eine solche Anordnung ermöglicht auch eine kompakte und schlanke Bauweise des Druckluftmotors, bzw. des ganzen Gerätes. Dies ist insbesondere bei Geräten, die in der Medizinaltechnik eingesetzt werden, sehr erwünscht und von grossem Vorteil.

Eine weitere vorteilhafte Ausführung besteht darin, dass im Bereich der Lamellen zwischen der Aussenseite des Rotors und der Führungsbohrung des Gehäuses eine die Aussenseite der Lamellen umhüllende, frei rotierbare Flughülse angeordnet ist, die an ihrem Mantel wenigstens eine am Umfang und/oder Ober die Länge der Flughülse unregelmässig verteilt angeordnete, als im wesentlichen radial verlaufende Bohrungen oder als im wesentlichen in Längsrichtung der Flughülse oder wendeiförmig verlaufende Schlitze ausgebildete Durchtrittsöffnung aufweist. Durch die Flughülse wird der Kontakt der Lamellen mit der Führungsbohrung des Gehäuses und somit ein Verschleiss der Lamellen an ihrer Aussenseite vermieden.

Die Flughülse und/oder die Lamellen bestehen zweckmässig aus Kunststoff, vorzugsweise aus Phenolharzgewebe. Solche Werkstoffe weisen ein relativ geringes spezifisches Gewicht, gute Temperaturbeständigkeit und günstige Reibungsverhältnisse auf.

Die Längsschlitze für die Lamellen sind vorteilhaft in den Radialebenen durch den Rotor angeordnet. Somit weist der Rotor in beiden Drehrichtungen gleiche Laufeigenschaften auf.

Zweckmässigerweise sind wenigstens zwei bis sechs, vorzugsweise vier Lamellen vorgesehen. Je höher die Anzahl der Lamellen ist, desto gleichmässiger ist das Laufverhalten und der Verlauf des Drehmomentes eines Druckluftmotors über eine Umdrehung.

### Figurenbeschreibung

Die Erfindung soll nachstehend, anhand der sie beispielsweise wiedergebenden Zeichnungen, näher erläutert werden. Es zeigen:
Fig. 1 einen erfindungsgemässen Mikro-Druckluftmotor, im Längsschnitt,
Fig.2 einen Querschnitt durch den in Fig. 1 dargestellten Mikro-Druckluftmotor, entlang der Linie A - A,
Fig. 3 einen Querschnitt durch den in Fig. 1 dargestellten Mikro-Druckluftmotor, entlang der Linie B - B, im Stillstand des Mikro-Druckluftmotors,
Fig. 4 einen Querschnitt durch den in Fig. 1 dargestellten Mikro-Druckluftmotor, entlang der Linie B - B, im Rechtslauf des MikroDruckluftmotors,
Fig. 5 einen Querschnitt durch den In Fig. 1 dargestellten Mikro-Druckluftmotor, entlang der Linie B - B, im Linkslauf des Mikro-Druckluftmotors.

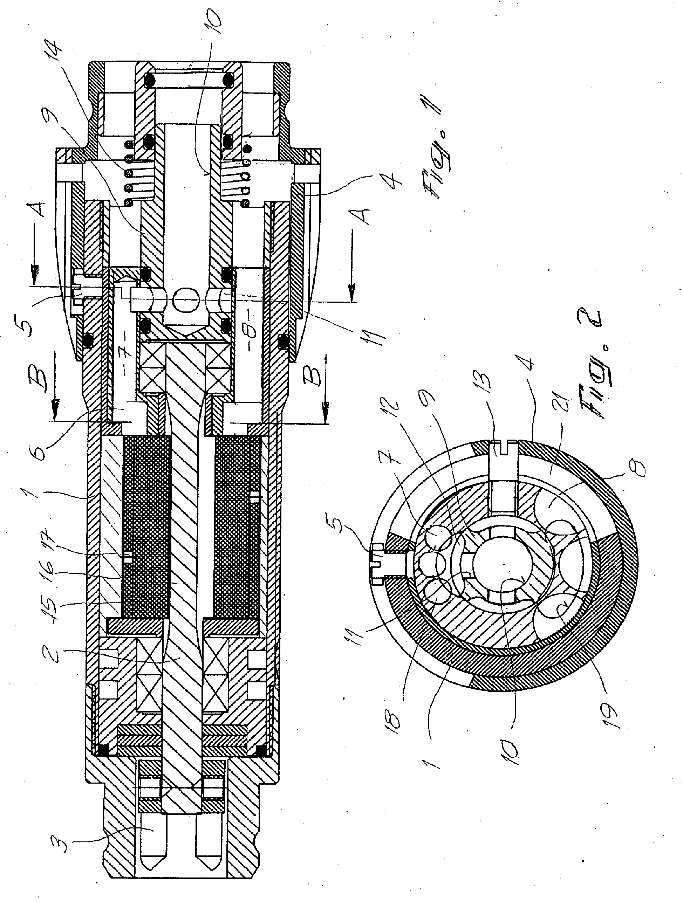

Der aus den Fig. 1 bis 5 ersichtliche Mikro-Druckluftmotor weist ein Gehäuse 1 mit einem darin drehbar gelagerten Rotor 2 auf. Das freie Ende des Rotors 2 ist mit einem Werkzeughalter 3 zur Aufnahme von auswechselbaren Werkzeugen verbunden. An seinem rückwärtigen Ende wird das Gehäuse 1 von einer Betätigungshülse 4 umgeben. Die Betätigungshülse 4 ist gegen die Kraft einer Feder 14 begrenzt axial verschiebbar und steuert dabei auch die Druckluftzufuhr. Ein Anschlag 5 begrenzt den axialen Verschiebeweg der Betätigungshülse 4. Im Gehäuse 1 ist ein Kollektor 6 begrenzt drehbar gelagert.

Der Kollektor 6 weist Längsbohrungen 7 und in axialer Richtung verlaufende, nach aussen hin offene Kanäle 8 für die Druckluftzufuhr und - abfuhr auf. Ein Adapter 9 ist mit dem Gehäuse 1 fest verbunden. Der Adapter 9 ist mit einer Zentralbohrung 10 sowie mit in die Zentralbohrung 10 mündenden Querbohrungen 11 versehen. Durch Ausfräsungen 12 wird die Verbindung zwischen den Querbohrungen 11 und den Längsbohrungen 7 des Kollektors 6 hergestellt.

Wie aus der Fig. 2 hervorgeht, ist die Betätigungshülse 4 über eine Stiftschraube 13 mit dem Kollektor 6 drehfest verbunden. Die Stiftschraube 13 greift durch einen über einen Teil des Umfanges verlaufenden Schlitz 21 des Gehäuses 1. Somit kann durch Verdrehen der Betätigungshülse 4 der damit gekoppelte Kollektor 6 ebenfalls verdreht werden. Die Endstellungen sind einerseits durch den Schlitz 21 im Gehäuse 1 und andererseits auch durch den Anschlag 5 begrenzt.

In Längsschlitzen 20 des Rotors 2 sind radial verschiebbar gelagerte Lamellen 15 angeordnet. Die Lamellen 15 werden an ihrer Aussenseite von einer Flughülse 16 umgeben. Die Flughülse 16 ist mit Durchtrittsöffnungen 17 für die Druckluft versehen. Somit besteht auf beiden Seiten der Flughülse 16 derselbe Druck und die Flughülse 16 ist dadurch fliegend und praktisch reibungsfrei gelagert. Die Längsbohrungen 7 munden in eine Eintrittsöffnung 18 und die Kanäle 8 in eine Austrittsöffnung 19.

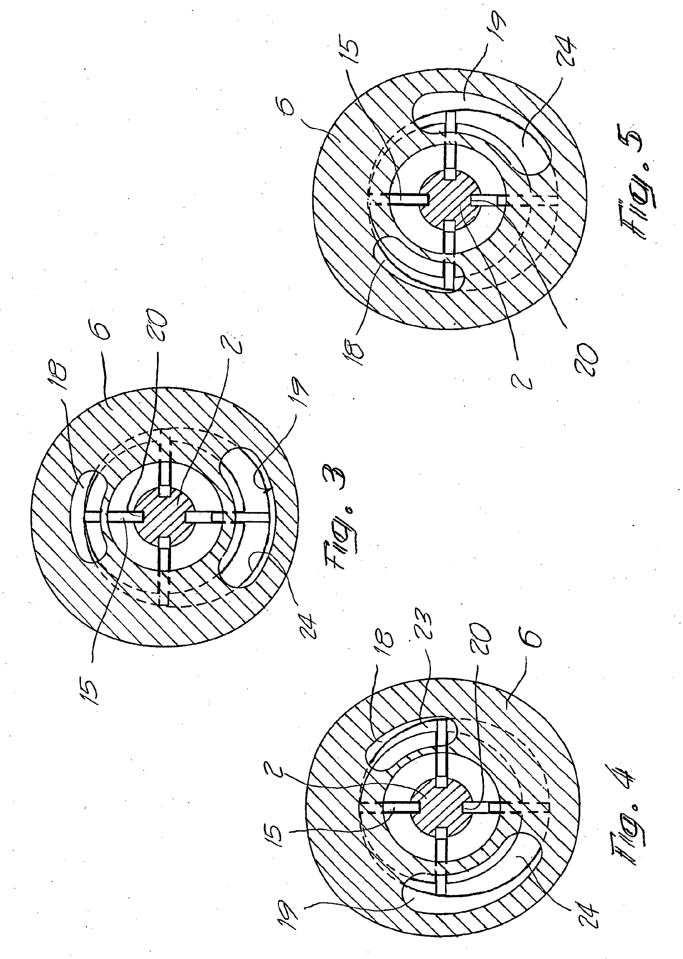

Fig. 3 zeigt den Kollektor 6 in der Neutralstellung entsprechend Fig. 2. Dabei wird die Lamelle 16 im Bereich der Eintrittsöffnung 18 von beiden Seiten her mit gleichem Druck beaufschlagt. Die Austrittsöffnung 19 auf der gegenüberliegenden Seite der Elntrittsöffnung 18 ist mit den Räumen auf beiden Seiten der Lamelle 15 verbunden. Der Rotor 2 ist in Winkeldrehstellung des Kollektors 6 stillgesetzt.

Bei der in Fig. 4 dargestellten Position ist der Kollektor 6 gegenüber der in Fig. 3 gezeigten Position um einen Winkel von ca. 60° im Uhrzeigersinn verdreht Die Eintrittsöffnung 18 ist nun mit der Arbeitsraum 23 verbunden, sodass durch den Luftdruck der Rotor 2 im Uhrzeigersinn rotiert Auf der Gegenseite mündet der Arbeitsraum 24 in die Austrittsöffnung 19. Wenn der Kollektor 6 in die in Fig. 3 dargestellte Ausgangsposition zurückverdreht wird, wird der Rotor 2 wiederum stillgesetzt.

Wird der Kollektor 6 im Gegenuhrzeigersinn bis in die in Fig. 5 dargestellte andere Endstellung verdreht, so rotiert der Rotor 2 aufgrund der umgekehrten Verhältnisse im Gegenuhrzeigersinn. Durch beliebige Zwischenstellungen zwischen den in Fig. 4 und 5 gezeigten Extrernstellungen und der in Fig. 3 dargestellten Neutralstellung kann die Drehzahl des Rotors 2 stufenlos reguliert werden.

Wie aus den Fig. 3 bis 5 ersichtlich ist, sind die Eintrittsöffnungen 18 und Austrittsöffnungen 19 im Querschnitt im Wesentlichen nierenförmig ausgebildet. Dadurch ist eine optimale strömungstechnische Anpassung im Übergangsbereich von der Eintrittsöffnung 18 und der Austrittsöffnung 19 zu den Arbeits-räumen 23 und 24 möglich.

### Bezugszeichenliste

- 1: Gehäuse
- 2: Rotor
- 3: Werkzeughalter
- 4: Betätigungshülle
- 5: Anschlag
- 6: Kollektor
- 7: Langsbohrung
- 8: Kanal
- 9: Adapter
- 10: Zentralbohrung
- 11: Querbohrung
- 12: Ausfräsung
- 13: Stiftschraube
- 14: Feder
- 15: Lamelle
- 16: Flughülse
- 17: Durchtrittsöffnung
- 18: Eintrittsöffnung
- 19: Austrittsöffnung
- 20: Längsschlitz
- 21: Schlitz
- 23: Arbeitsraum
- 24: Arbeitsraum

## Patentansprüche

1. Mikro-Druckluftmotor mit einem eine Führungsbohrung aufweisenden Gehäuse (1) und einem darin drehbar gelagerten Rotor (2), wobei der Rotor mit im wesentlichen radial nach aussen verlaufenden Schlitzen versehen ist, in denen plattenförmige Lamellen (15) durch die Fliehkraft radial nach aussen verschiebbar gelagert sind, und mit einem axial an den Rotor anschliessenden Kollektor (6) mit zumindest je einer Ein- und Austrittsöffnung (18, 19) und mit äusseren Anschlüssen für die Druckluft verbundenen und in die zumindest. eine Austrittsöffnung mündenden Kanälen (8) und mit einer Betätigungschülse (4) wobei der Kollektor (6) mittels der Betätigungshülse (4) um die Drehachse des Rotors (2) oder um eine dazu parallele Achse, bzw. um die Längsachse des Gehäuses (1) aus einer zentralen, den Rotor (2) stillsetzenden Neutralstellung nach beiden Seiten hin bis in eine den Rotor (2) unter Druckluft links- bzw. rechtsdrehende Endstellung verdrehbar ist, **dadurch gekennzeichnet, dass** in Neutralstellung des Kollektors (6) die Räume auf beiden Seiten der mit Druck beaufschlagten Lamelle (15) mit zumindest einer Eintrittsöffnung (18) derart verbunden sind, dass die Lamelle (15) im Bereich der Eintrittsöffnung (18) von beiden Seiten her mit gleichem Druck beaufschlagt wird.

2. Mikro-Druckluftmotor nach Anspruch 1, **dadurch gekennzeichnet, dass** in Neutralstellung des Kollektors (6) die Räume auf beiden Seiten der Lamelle (15), welche der mit Druck beaufschlagten Lamelle (15) gegenüberliegt, mit zumindest einer Austrittsöffnung (19) verbunden sind.

3. Mikro-Druckluftmotor nach Anspruch 1 oder 2, **dadurch gekennzeichnet, dass** zumindest eine der Eintrittsöffnungen (18) und/oder der Austrittsöffnungen (19) mit nierenförmige Querschnitt und zur Drehachse des Rotors (2) hin gekrümmt ausgeführt ist.

4. Mikro-Druckluftmotor nach einem der Ansprüche 1 bis 3, **dadurch gekennzeichnet, dass** die Ein- und Austrittsöffnungen (18,19) unterschiedliche Querschnitte aufweisen.

5. Mikro-Druckluftmotor nach zumindest einem der Ansprüche 1 bis 4, **dadurch gekennzeichnet, dass** der Kollektor (6) stufenlos bis zu den Endstellungen verdrehbar ist.

6. Mikro-Druckluftmotor nach zumindest einem der Ansprüche 1 bis 5, **dadurch gekennzeichnet, dass** der Kollektor (6) mit der Betätigungshülse (4) gekoppelt ist.

7. Mikro-Druckluftmotor nach zumindest einem der Ansprüche 1 bis 6, **dadurch gekennzeichnet, dass** wenigstens ein Kanal (7, 8) koaxial, bzw. parallel zur Achse des Rotors (2) verläuft.

8. Mikro-Druckluftmotor nach zumindest einem der Ansprüche 1 bis 7, **dadurch gekennzeichnet, dass** im Bereich der Lamellen (15) zwischen der Aussenseite des Rotors (2) und der Führungsbohrung des Gehäuses (1) eine die Aussenseite der Lamellen (15) umhüllende, frei rotierbare Flughülse (16) angeordnet ist, die an ihrem Mantel wenigstens eine am Umfang und/oder über die Länge der Flughülse (16) unregelmässig verteilt angeordnete, als im wesentlichen radial verlaufende Bohrungen oder als im wesentlichen in Längsrichtung der Flughülse (16) oder wendelförmig verlaufende Schlitze ausgebildete Durchtrittsöffnung (17) aufweist.

9. Mikro-Druckluftmotor nach Anspruch 8. **dadurch gekennzeichnet, dass** die Flughülse (16) und/oder die Lamellen (15) aus Kunststoff, vorzugsweise aus Phenolharzgewebe, bestehen.

10. Mikro-Druckluftmotor nach zumindest einem der Ansprüche 1 bis 9, **dadurch gekennzeichnet, dass** die Längsschlitze (20) für die Lamellen (15) in den Radialebenen durch den Rotor (2) angeordnet sind.

11. Mikro-Druckluftmotor nach zumindest einem der Ansprüche 1 bis 10, **dadurch gekennzeichnet, dass** wenigstens zwei bis sechs, vorzugsweise vier Lamellen (15) vorgesehen sind.

## Claims

1. A micro-compressed-air motor, having a housing (1) having a guide hole and a rotor (2) rotatably mounted therein, wherein the rotor is provided with essentially radially outwardly running slots, in which plate-shaped slats (15) are mounted such that they can be displaced radially outwards by centrifugal force, and having a collector (6), which can be axially connected to the rotor, having at least one of each of an inlet and outlet opening (18, 19) and having channels (8) connected to external connectors for the compressed air and opening into the at least one outlet opening and having an actuation sleeve (4), wherein the collector (6) can be rotated by means of the actuation sleeve (4) about the axis of rotation of the rotor (2) or about an axis parallel thereto, or about the longitudinal axis of the housing (1), out of a central neutral position, which stops the rotor (2), to both sides up to an end position rotating the rotor (2) anti-clockwise or clockwise under compressed air, **characterised in that** in the neutral position of the collector (6), the spaces on both sides of the pressure-loaded slat (15) are connected to at least one inlet opening (18) in such a manner that the slat (15) is loaded with equal pressure from both sides in the region of the inlet opening (18).

2. The micro-compressed-air motor according to Claim 1, **characterised in that** in the neutral position of the collector (6), the spaces on both sides of the slat (15), which lies opposite the pressure-loaded slat (15), are connected to at least one outlet opening (19).

3. The micro-compressed-air motor according to Claim 1 or 2, **characterised in that** at least one of the inlet openings (18) and/or the outlet openings (19) is realised with a kidney-shaped cross section and curved towards the axis of rotation of the rotor (2).

4. The micro-compressed-air motor according to one of Claims 1 to 3, **characterised in that** the inlet and outlet openings (18, 19) have different cross sections.

5. The micro-compressed-air motor according to at least one of Claims 1 to 4, **characterised in that** the collector (6) can be rotated in an infinitely variable manner as far as the end positions.

6. The micro-compressed-air motor according to at least one of Claims 1 to 5, **characterised in that** the collector (6) is coupled to the actuation sleeve (4).

7. The micro-compressed-air motor according to at least one of Claims 1 to 6,**characterised in that** at least one channel (7, 8) runs coaxially or parallel to the axis of the rotor (2).

8. The micro-compressed-air motor according to at least one of Claims 1 to 7, **characterised in that** a freely rotatable floating sleeve (16), which surrounds the exterior of the slats (15), is arranged in the region of the slats (15) between the exterior of the rotor (2) and the guide hole of the housing (1), which floating sleeve has at least one through-hole (17) on the jacket thereof, arranged in an irregularly distributed manner on the circumference and/or over the length of the floating sleeve (16), realised as essentially radially running holes or constructed as slots running essentially in the longitudinal direction of the floating sleeve (16) or running helically.

9. The micro-compressed-air motor according to Claim 8, **characterised in that** the floating sleeve (16) and/or the slats (15) consist of plastic, preferably of phenolic resin fabric.

10. The micro-compressed-air motor according to at least one of Claims 1 to 9, **characterised in that** the longitudinal slots (20) for the slats (15) are arranged through the rotor (2) in the radial planes.

11. The micro-compressed-air motor according to at least one of Claims 1 to 10, **characterised in that** at least two to six, preferably four slats (15) are provided.

## Revendications

1. Micromoteur à air comprimé avec un boîtier (1) présentant un alésage de guidage et un rotor (2) logé de manière rotative à l'intérieur, le rotor étant muni de fentes s'étendant sensiblement de manière radiale vers l'extérieur dans lesquelles des lamelles en forme de plaques (15) sont logées de manière à pouvoir coulisser radialement vers l'extérieur du fait de la force centrifuge, et avec un collecteur (6) en jonction axiale au rotor avec au moins respectivement une ouverture d'entrée et de sortie (18, 19) et avec des canaux (8) en liaison avec des raccords externes pour l'air comprimé et débouchant dans l'au moins une ouverture de sortie et avec un manchon d'actionnement (4), moyennant quoi on peut faire tourner le collecteur (6) à l'aide du manchon d'actionnement (4) autour de l'axe de rotation du rotor (2) ou autour d'un axe parallèle à celui-ci, ou autour de l'axe longitudinal du boîtier (1) à partir d'une position centrale neutre arrêtant le rotor (2) vers le deux côtés jusqu'à une position finale faisant tourner le rotor (2) vers la gauche ou la droite sous l'effet de l'air comprimé, **caractérisé en ce que**, dans la position neutre du collecteur (6), les espaces des deux côtés de la lamelle (15) sur laquelle une pression s'exerce sont reliés à au moins une ouverture d'entrée (18) de manière à ce que, dans la zone de l'ouverture d'entrée (18), la lamelle (15) est soumise à une pression identique depuis les deux côtés.

2. Micromoteur à air comprimé selon la revendication 1, **caractérisé en ce que**, dans la position neutre du collecteur (6), les espaces des deux côtés de la lamelle (15) qui est située en vis-à-vis de la lamelle (15) sur laquelle une pression s'exerce, sont reliés à au moins une ouverture de sortie (19).

3. Micromoteur à air comprimé selon la revendication 1 ou 2, **caractérisé en ce que** au moins l'une des ouvertures d'entrée (18) et/ou des ouvertures de sortie (19) est réalisée avec une section transversale en forme de rein et de manière courbée vers l'axe de rotation du rotor (2).

4. Micromoteur à air comprimé selon l'une des revendications 1 à 3, **caractérisé en ce que** les ouvertures d'entrée et de sortie (18, 19) présentent des sections transversales différentes.

5. Micromoteur à air comprimé selon l'une au moins des revendications 1 à 4, **caractérisé en ce que** l'on peut faire tourner le collecteur (6) en continu jusqu'aux positions finales.

6. Micromoteur à air comprimé selon l'une au moins des revendications 1 à 5, **caractérisé en ce que** le collecteur (6) est couplé avec le manchon d'actionnement (4).

7. Micromoteur à air comprimé selon l'une au moins des revendications 1 à 6, **caractérisé en ce qu'**au moins un canal (7, 8) s'étend de manière coaxiale ou parallèle à l'axe du rotor (2).

8. Micromoteur à air comprimé selon l'une au moins des revendications 1 à 7, **caractérisé en ce que**, dans la zone des lamelles (15), entre le côté extérieur du rotor (2) et l'alésage de guidage du boîtier (1), une gaine flottante (16) pouvant tourner librement, enveloppant le côté extérieur des lamelles (15), est disposée, laquelle présente sur son enveloppe au moins une ouverture de passage (17) disposée de manière irrégulièrement répartie sur le pourtour et/ou sur la longueur de la gaine flottante (16) en tant qu'alésages s'étendant sensiblement radialement ou bien en tant que fentes s'étendant sensiblement en direction longitudinale de la gaine flottante (16) ou en forme d'hélice.

9. Micromoteur à air comprimé selon la revendication 8, **caractérisé en ce que** la gaine flottante (16) et/ou les lamelles (15) se composent de plastique, de préférence d'un tissu en résine phénolique.

10. Micromoteur à air comprimé selon l'une au moins des revendications 1 à 9, **caractérisé en ce que** les fentes longitudinales (20) pour les lamelles (15) sont disposées dans les plans radiaux à travers le rotor (2).

11. Micromoteur à air comprimé selon l'une au moins des revendications 1 à 10, **caractérisé en ce que** l'on prévoit au moins deux à six, de préférence quatre lamelles (15).
